# EUROPEAN PATENT SPECIFICATION

(11) **EP 0 262 760 B1**
(45) Date of publication and mention of the grant of the patent: **27.01.1993**
(21) Application number: 87305963.8
(22) Date of filing: 06.07.1987
(51) Int. Cl.: G01N 33/53, G01N 33/543, G01N 33/553, G01N 33/557

(54) **Method and apparatus for immunoassay**
Verfahren und Apparat für Immunoassay
Méthode et appareil pour essai immunologique

(30) Priority: 04.07.1986 JP 157605/86
(43) Date of publication of application: 06.04.1988
(73) Proprietor: Tosoh Corporation, Shinnanyo-shi, Yamaguchi-ken, 746 (JP)
(72) Inventor: Kamada, Satoru, Yamato-shi Kanagawa-ken (JP); Iwasaki, Shuji, Machida-shi Tokyo (JP)
(74) Representative: Sheard, Andrew Gregory

(56) References cited:
- EP-A- 0 177 813
- EP-A- 0 180 384
- EP-A- 0 211 436
- EP-A- 0 216 177
- DE-A- 2 905 434

## Description

The present invention relates to an assay method for measuring immune reactions and to an apparatus which can be used to detect a very small amount of material of organisms.

As immunological means for detecting a very small amount of material of organisms, a great number of studies and propositions on assay methods for detecting immune reactions have been recently made, including radioimmunoassay, fluorescent antibody method, enzyme antibody method, enzymeimmunoassay, etc.

In these test methods representatively described, either an antigen or an antibody (or antiimmunoglobulin), which undertakes a specific reaction, is conjugated with an appropriate marker, the complex of the antigen-antibody-marker or antibody-antigen-marker is formed, and the amount of the complex is quantitatively detected by measuring the marker as a criterion. The complex is formed by binding to the surface of a cell on which either an antigen or an antibody is fixed, or to the surface of a bead (particle), which is charged into a cell, on which either an antigen or antibody is fixed.

In other methods apart from a radioimmunoassay using a radioactive substance as a marker, a fluorescent substance or a luminescent substance used as a marker is directly detected optically, or the catalytic activity of enzyme used as a marker is then revealed by the addition of an appropriate substrate and absorbance, fluorescence or luminescence is determined optically.

In various means for optical determination, the principle is that a microtiter plate serving as a cell or a sample reaction chamber, or a reactor (referred to as a cell hereinafter) composed of a separate test cup is placed opposite to the optical system, which received reflected light from the cell, for use in determination of the intensity of light.

Since the amount of samples to be tested in immune reactions is extremely small, usually less than 10⁻¹³ mol/l, factors causing technical errors in the test should be excluded as much as possible even if a strictly precise equipment would be employed in the aforesaid optical system.

EP86111834.7/EP-A-0216177 and EP86110787.8/EP-A-0211436 belong to the prior art under Article 54(3)EPC.

The present invention measures the concentration (amount) of the object to be measured by measuring the signal from within a limited area of the cell while the beads are being vibrated.

In EP216177 the fluorescente is measured while the beads are being vibrated in a reaction liquid having an adjusted pH range suitable for enzyme reaction to measure the enzyme concentration (amount) from the fluorescente increase (rate). EP211436 is concerned with stirrers for biochemical reactions involving vibrating beads but does not disclose the use of such a stirrer in rate assays.

The present inventors made various investigations from these points of view, and obtained a knowledge that in the test method using a bead, which has been known as a method for detecting the immune reaction, beads existing in the cell affected technical errors in test; that is, since the measuring area in the optical system which receives the reflected light from the cell is, in general, limited in the part within the cell, the existence of the bead, in short, whether or not the bead exists within the measuring area of the optical system, is considered to have an influence on measurement.

According to the present invention an immunoassay for the determination of a first component, the assay comprising causing a second component capable of forming an antigen-antibody complex with the first component to form such a complex, one of the first and second components being linked directly or indirectly to a bead within a cell, and the other of the first and second components being linked directly or indirectly to a marker which is quantitatively determinable by optical measurements, and quantitatively determining the amount of marker the bead being vibrated during optical measurement, is characterised in that the optical measurement is carried out in a limited area of the cell.

From those points of view, in the test method where immune reactions are performed on the surface of a bead in a cell, the present invention was made to obtain an effective method, which makes technical errors in optical determination as small as possible, and to supply an apparatus fulfilling the requirements.

One embodiment of the present invention is to form a complex of the enzyme conjugate due to the antigen-antibody reaction on the surface of the bead filled in a cell (i.e. the complex of the enzyme labeled antibody or antigen with an antigen, antibody, or antigen-antibody), and to detect the amount of the complex by optical means with vibrating the beads within a cell. An example of an apparatus according to this embodiment is to be composed of a cell which supplies a reacting chamber where the antigen-antibody reaction takes place, the bead which offers the binding surface to an antigen or antibody in the cell and contains magnetic substance, a magnet equipment which has a magnetic action on the bead from the outside of the cell and allows it vibrate, and an optical system by which the intensity of the reflected light from the inside of the cell is measured.

The vibration of beads within the cell according to the present invention will lead to level the existence probability of beads in the limited area for optical measurement within the cell and to lose technical errors derived from the partial existence of beads. The frequency of vibration of beads, therefore, ranges usually from 10 rpm to 800 rpm, preferably 60 rpm to 360 rpm, and the amplitude of vibration is desired to be enough to spread over the whole area in the cell. Any vibration, including reciprocating motion, circular motion, elliptic motion, and S-curve motion, can be permitted.

Vibration of the bead alone within the cell in the present invention was based on the fact that the mechanical vibration of the test cup itself as a cell will cause a liquid sample to scatter outside of the cell and also inadequate vibration of beads.

The bead used in one apparatus according to the present invention contains a magnetic substance, preferably a paramagnetic substance, migrating by the magnetic action which produces vibration. Preferably, the powder of magnetic substance such as Mn-Zn-ferrite is bound to the beads with the synthetic resin binders, such as polystyrene and EVA, the surface of beads possessing such functional groups as amino, hydroxy,epoxy, and aldehyde by polymerizing such a glycidylmethacrylate polymer. Binding of antigens or antibodies to the surface of the bead may be carried out by the known method.

The magnetic apparatus generating the magnetic action, by which the bead vibrates, may e.g. be composed of the following two mechanisms; in short, one is a reciprocating motion of a rod, to which a magnet is fixed, close to the position where the test cell is placed, and the other is to produce the magnetic field using an electromagnet.

The present invention can be applied to various immunoreaction methods using beads and measuring optically, examples being a method in which a fluorescent substances (fluoresceine, etc.) or luminescent substances (isoluminole, luminole, etc.) are used as a marker and the intensity of light is measured, and another method in which enzymes such as β-D-galactosidase, alkaline phosphatase, glucoseoxidase, and peroxidase are used as a marker and the fluorescence or absorbance of a substrate on receiving the action of the enzyme activity is measured.

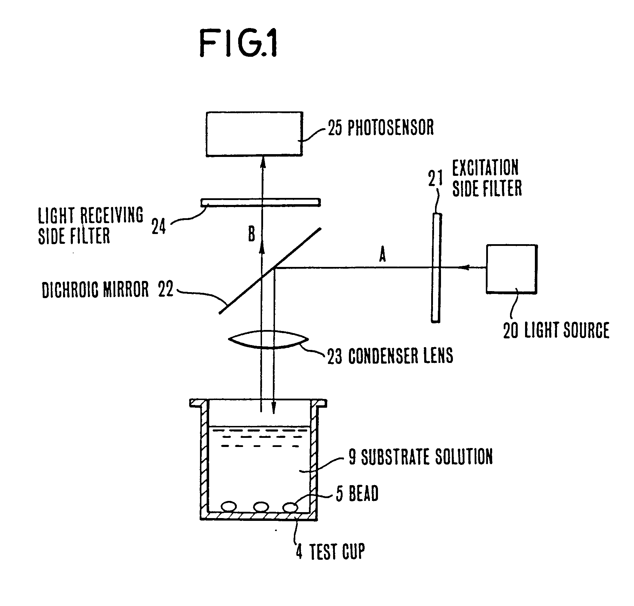

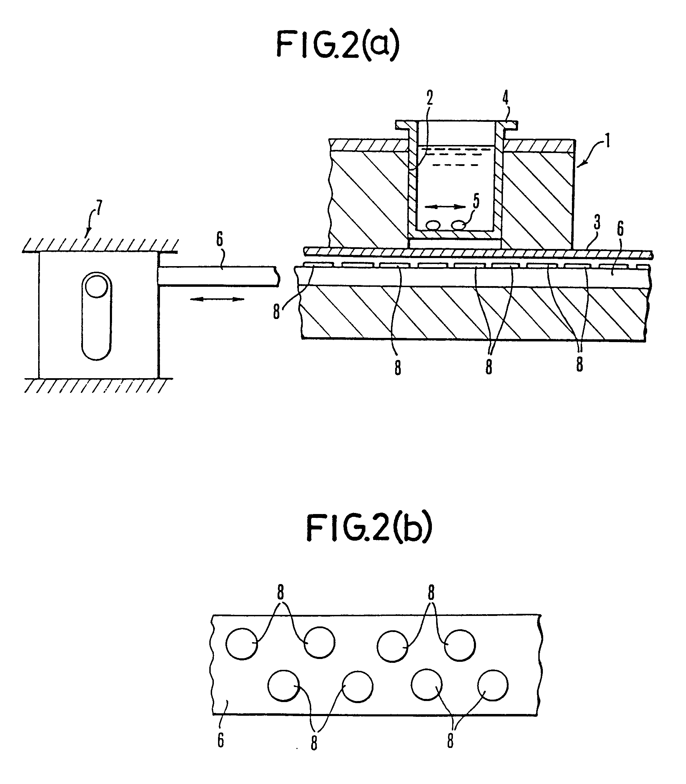

Further objects and features of the present invention will become apparent from the following detailed description of the preferred embodiment thereof with reference to the accompanying drawings, in which
Fig. 1 shows the outline of constituents of the optical measuring apparatus explaining the embodiment of the present invention. Figs. 2(a) and 2(b) are expanded figures of the constituents of the bead vibrating apparatus.

The following will describe an embodiment of the present invention according to the drawings.

Fig. 1 shows the outline of constituents of the optical measuring system. Figs. 2(a) and 2(b) are the outline of constituents of the magnet apparatus functioning as a vibrating magnet.

In the drawings, 1 is a test plate which can be carried on the conveyance way 3 and has a large number of opening 2 for use in supporting a cup.

4 is a test cup inserted and fixed to the opening 2; in the present embodiment, it is made of magnetic permission and opaque obtained from the polystyrene resin containing graphite, as a cylinder cup in the upper opened type, and serves as a cell which is a reacting chamber.

5 is a bead in the cup 4, which is containing magnetic substance and at the surface of which the first antibody which specifically binds to the sample to be tested has been bound according to the known method.

6 is a rod placed under the conveyance way 3 where the test plate 1 is put on, and it is reciprocated at a given stroke in the direction indicated by the arrow in Fig. 2(a) due to a cam mechanism 7 which is rotated by the drive motor (not shown in the figure). And on this rod 6, a large number of magnets 8 as corresponding to each test cup 4 are fixed as shown in the figure.

According to the reciprocating motion of the rod 6, therefore, beads 5 within the test cup 4 will migrate in the same direction as in the circular motion, elliptic motion, and S-curve motion.

Against the test cup 4, the optical measuring apparatus, e.g. the well-known microplate fluorescence automatical reader, as schematically shown in Fig. 1, is placed at the upper side, letting the light A in from the light source and measuring a radiating fluorescence B.

That is, the well-known optical measuring apparatus, as shown in Fig. 1, operates as follows:
To the test cup containing beads 5, on the surface of which the enzyme-linked conjugates are bound due to the specific immune reaction, and an appropriate substrate solution 9 which causes optically detectable changes due to the aforesaid enzyme activity, the light from the light source 20 travels through the excitation side filter 21 via a dichroic mirror 22 and the condenser lens 23 enters the test cup 4, and then the reflected light from the test cup 4 proceeds via the condenser lens 23, the dichroic mirror 22 and the light receiving side filter 24, and the light is received by the photosensor 25, and at the signal processing circuit (not shown) the intensity of detected light will be measured.

In such a constitution, according to the known enzyme immunoassay methods, the complex of the first antibody (anti-HCG) on the solid beads - antigen (HCG) - enzyme (alkaline phosphatase) conjugated second antibody (anti-HCG) is formed on the surface of beads, and after the free enzyme conjugated second antibody is removed by B/F separation, a substrate solution (4-methylumbelliferylphosphate monoester) which generates fluorescence by the action of the aforesaid enzyme is added, and changes in the intensity of fluorescence occurring in the substrate is measured with vibrating beads 5 by reciprocating motion of the rod 6.

In such a procedure, since the existence probability of vibrating beads to the limited area for optical measurement within the test cup is levelled on measuring the light, technical errors will be dissolved which has been so far made by the existence of beads within the cell.

The present test method, further, has such an effect that vibration of beads produces agitation of the substrate within the cell, hence apparent increase in the intensity of fluorescence of a substrate due to the enzyme activity occurs in directly proportional to the amount of enzyme, and also has an advantage for more accurate quantitative measurement of enzyme and antigen in case that the rate of increase in the intensity of fluorescence is measured as a criterion.

### Example :

Ferritin was used as a material to be measured and 12 pieces of bead, about 1 mm in diameter, were put in a cell, 8 mm in inner diameter.

Magnets 8, each of which is a round rare-earth magnet, 5 mm in a diameter and 3 mm in thickness, fixed on the rod 6, which is used for vibrating beads are placed at the pitch of 16 mm and in double lines (the distance between the centers of the magnets in the right and left lines is 5 mm), as shown in Fig. 2(b).

In the first reaction, the antigen-antibody reaction was made for 40 min. with vibrating (stroke 48 mm, 80 rpm) the magnet apparatus, and after the B/F separation the enzyme substrate solution was added, and changes in the amount of fluorescence (4 methylumbelliferone) decomposed by enzyme under both vibrating and not vibrating, an experimental control, was measured as the rate of increase in the intensity of fluorescence.

Test results were indicated in Tables 1 and 2. In the determination under vibration, the reproducibility (coefficient of variation CV %) in ten times of determination was 7.08 % at 0 concentration of ferritin, 4.45 % at a lower concentration (L) (about 50 ng/ml), 4.40 % at a medium concentration (M) (500 ng/ml), and 3.70 % at a higher concentration (H) (about 800 ng/ml).

On the contrary, in the determination under not vibration, the reproducibility was extremely low, 29.53 %, 18.26 %, 19.11 % and 10.14 %, respectively.

Under no vibration, further, the rate of production of fluorescence was small at a high concentration and its linear state was not observed as indicated in vibration. These results are based on the situation that the diffusion of substrate at the surface of beads was inadequate.

These results indicate that technical errors in optical determination decrease and high reproducibility can be obtained under a given conditions.

Incidentally, the present embodiment presented a test method measuring the fluorescence intensity of a substrate as an enzyme immunoassay, but this test method may be substituted for the method measuring the absorbance of the substrate, and also for that using a fluorescent substance or a luminescent substance instead of an enzyme as a marker. In any of these methods, effects based on that the probability of existence of a bead in a limited area for optical determination within the cell is levelled by vibrating beads can be obtained.

The present invention, as mentioned above, helps solve the problem of technical errors in optical determination derived from the existence of a bead by vibrating the bead within the cell and lead the improvement in accuracy of determination, in general, where an extremely small amount of material is quantitatively dealt with; their advantages will render great service in immunoassay methods.

While a specific embodiment of the present invention has been shown and described in detail to illustrate the application of the inventive principles, it will be understood that the invention may be embodied otherwise without departing from such principles.

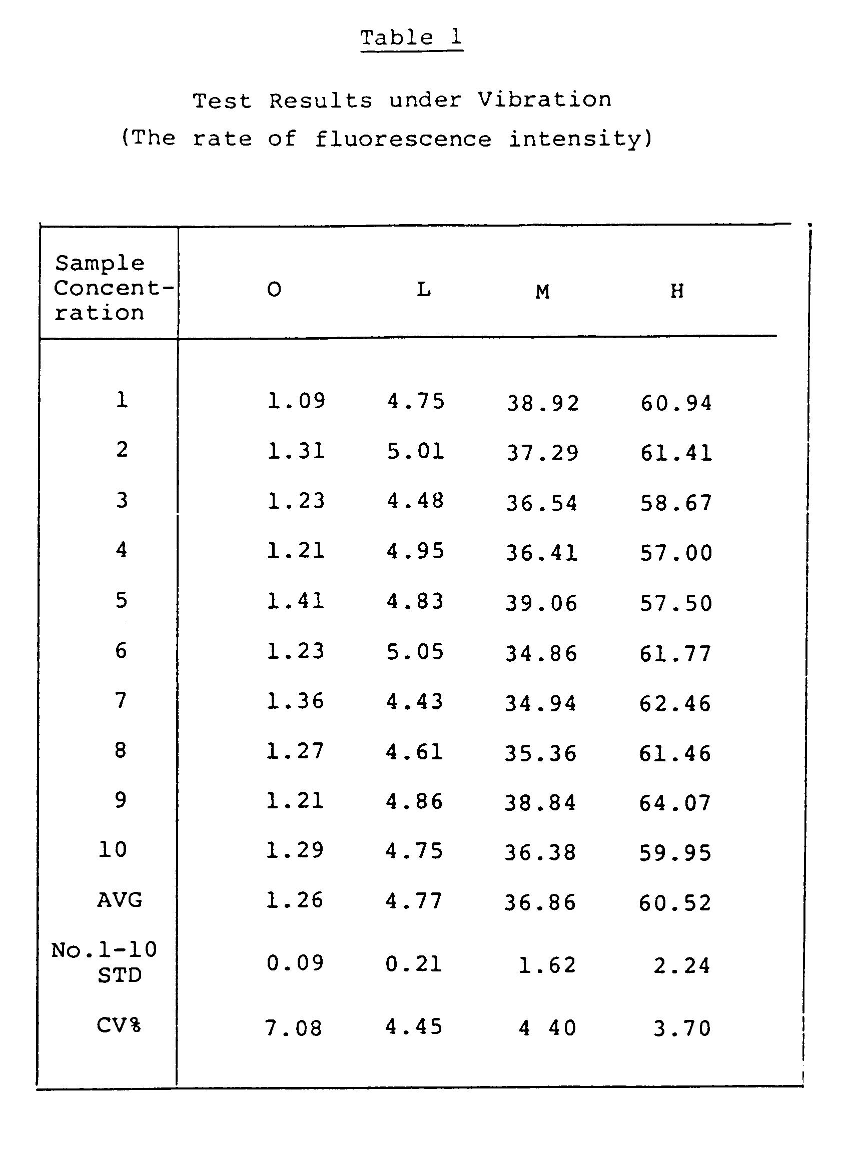

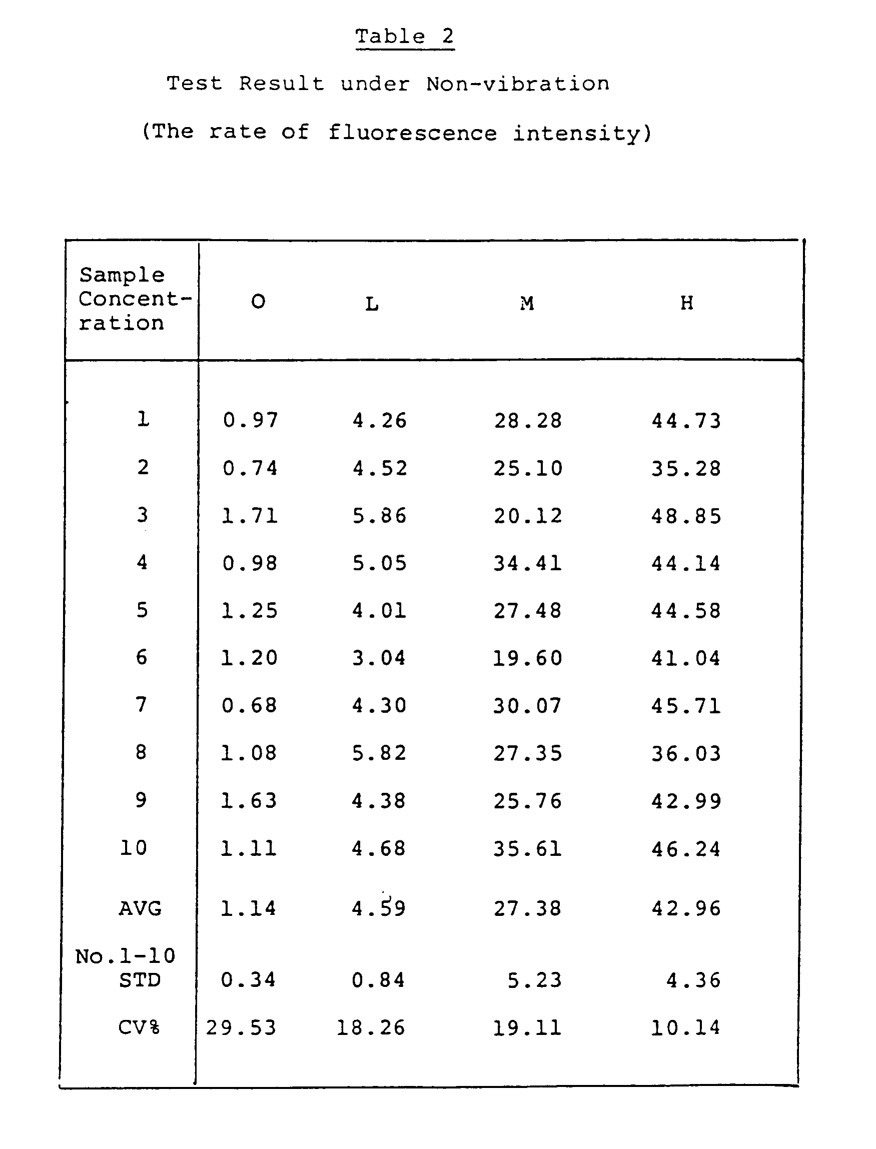

## Claims

1. An immunoassay for the determination of a first component, the assay comprising causing a second component capable of forming an antigen-antibody complex with the first component to form such a complex, one of the first and second components being linked directly or indirectly to a bead within a cell, and the other of the first and second components being linked directly or indirectly to a marker which is quantitatively determinable by optical measurements, and quantitatively determining the amount of marker, the bead being vibrated during optical measurement, characterised in that the optical measurement is carried out in a limited area of the cell.

2. A method as claimed in Claim 1, wherein the marker is an enzyme.

3. A method as claimed in Claim 1 or Claim 2 wherein the vibration is effected magnetically.

4. An apparatus suitable for use in carrying out an immunoassay as defined in anyone of Claims 1 to 3, the apparatus comprising a reaction cell, a bead within the cell to which the said one of the first and second components can be linked and optical measurement means for determining the amount of marker, and means for causing the bead to vibrate, characterised in that the optical measurement means are arranged to carry out the optical measurement in a limited area of the cell.

5. An apparatus as claimed in Claim 4 wherein the bead comprises a magnetic substance and the vibration means magnetically causes the bead to vibrate.

6. An apparatus as claimed in Claim 5 wherein the magnetic substance is paramagnetic.

## Patentansprüche

1. Immuntest zur Bestimmung einer ersten Komponente, wobei der Test umfaßt, Bewirken, daß eine zweite Komponente, die zur Bildung eines Antigen-Antikörperkomplexes mit der ersten Komponente befähigt ist, einen derartigen Komplex bildet, wobei entweder die erste oder die zweite Komponente direkt oder indirekt an ein Teilchen innerhalb der Zelle gebunden ist und die andere der ersten oder zweiten Komponente direkt oder indirekt an einen Marker gebunden ist, der sich quantitativ durch optische Messungen bestimmen läßt, und quantitatives Bestimmen der Menge an Marker, wobei das Teilchen während der optischen Messung in Vibrationen versetzt wird, **dadurch gekennzeichnet**, daß die optische Messung in einem begrenzten Bereich der Zelle durchgeführt wird.

2. Verfahren nach Anspruch 1, wobei der Marker ein Enzym ist.

3. Verfahren nach Anspruch 1 oder Anspruch 2, wobei die Vibration magnetisch bewirkt wird.

4. Vorrichtung, geeignet zur Verwendung in der Durchführung eines Immuntests nach einem der Ansprüche 1 bis 3, wobei die Vorrichtung eine Reaktionszelle, ein Teilchen innerhalb der Zelle, an das entweder die erste oder die zweite Komponente gebunden werden kann und optische Meßeinrichtungen zur Bestimmung der Menge an Marker und eine das Vibrieren des Teilchens bewirkende Einrichtung aufweist, **dadurch gekennzeichnet**, daß die optischen Meßeinrichtungen so angeordnet sind, daß die optische Messung in einem begrenzten Bereich der Zelle durchgeführt wird.

5. Vorrichtung nach Anspruch 4, wobei das Teilchen eine magnetische Substanz aufweist und die Vibrationseinrichtung das Teilchen magnetisch zum Vibrieren bringt.

6. Vorrichtung nach Anspruch 5, wobei die magnetische Substanz paramagnetisch ist.

## Revendications

1. Immunodosage pour la détermination d'un premier composant, l'analyse consistant à faire en sorte qu'un second composant puisse former un complexe antigène-anticorps avec le premier composant, et pour former un tel complexe, soit le premier, soit le second composant étant lié directement ou indirectement à une perle au sein d'une cellule, l'autre composant étant lié directement ou indirectement à un marqueur quantitativement déterminable par des mesures optiques, et à déterminer par voie quantitative la quantité de marqueur alors que la perle est soumise à des vibrations au cours de la mesure optique, caractérisé en ce que la mesure optique est effectuée dans une zone limitée de la cellule.

2. Procédé selon la revendication 1, dans lequel le marqueur est une enzyme.

3. Procédé selon la revendication 1 ou 2, dans lequel on réalise la vibration par voie magnétique.

4. Appareil approprié pour être utilisé dans la mise en oeuvre d'un immunodosage tel que défini dans l'une quelconque des revendications 1 à 3, l'appareil comprenant une cellule réactionnelle, une perle au sein de la cellule, à laquelle on peut lier un desdits premier et second composants, ainsi qu'un moyen de mesure optique pour déterminer la quantité de marqueurs, et un moyen pour provoquer la vibration de la perle, caractérisé en ce que les moyens de mesure optique sont disposés pour effectuer la mesure optique dans une zone limitée de la cellule.

5. Appareil selon la revendication 4, dans lequel la perle comprend une substance magnétique et le moyen de vibration provoque la vibration de la perle par voie magnétique.

6. Appareil selon la revendication 5, dans lequel la substance magnétique est une substance paramagnétique.
